(19) Europäisches Patentamt
European Patent Office
Office européen des brevets

(11) **EP 0 790 045 B1**

(12) **FASCICULE DE BREVET EUROPEEN**

(45) Date de publication et mention de la délivrance du brevet:
**15.01.2003 Bulletin 2003/03**

(51) Int Cl.⁷: **A61F 2/36**

(21) Numéro de dépôt: **97400191.9**

(22) Date de dépôt: **28.01.1997**

(54) **Tige d'ancrage utilisée notamment dans les prothèses**

Verankerungsschaft, insbesondere zur Verwendung in Prothesen

Anchoring shaft, especially for use in prostheses

(84) Etats contractants désignés:
**AT CH DE ES FR GB IT LI**

(30) Priorité: **08.02.1996 FR 9601541**

(43) Date de publication de la demande:
**20.08.1997 Bulletin 1997/34**

(73) Titulaire: **Deckner, André Georges**
**F-75015 Paris (FR)**

(72) Inventeur: **Deckner, André Georges**
**F-75015 Paris (FR)**

(74) Mandataire: **Eidelsberg, Victor Albert et al**
**Cabinet Flechner**
**22, Avenue de Friedland**
**75008 Paris (FR)**

(56) Documents cités:
**EP-A- 0 159 510**   **EP-A- 0 222 236**
**EP-A- 0 382 429**   **EP-A- 0 715 835**
**DE-U- 8 811 758**   **DE-U- 9 402 934**
**FR-A- 2 528 307**   **FR-A- 2 602 672**
**FR-A- 2 641 462**   **FR-A- 2 673 833**

## Description

Au DE 9 402 934, on décrit une tige d'ancrage suivant le préambule de la revendication 1.

Une tige d'ancrage doit bien s'immobiliser lors de sa mise en place (immobilisation primaire) et au fil du temps ne doit pas s'enfoncer de plus en plus dans le canal médullaire de l'os ni prendre du jeu.

L'invention vise une tige d'ancrage qui est douée de ces deux propriétés.

La tige d'ancrage suivant l'invention est définie à la revendication 1.

Comme les arêtes sont tranchantes, elles pénètrent bien dans l'os lors de leur introduction, ce qui assure une bonne immobilisation primaire.

Mais, comme l'angle entre les deux faces les plus éloignées des dièdres de chaque paire d'arête a les valeurs indiquées ci-dessus, la surface face à l'os augmente plus que proportionnellement au fur et à mesure que la tige commence à s'enfoncer dans l'os. On atteint rapidement un équilibre dans lequel l'os résiste à la tendance à la prothèse à s'enfoncer.

Les arêtes s'étendent sensiblement sur toute la longueur de la zone d'ancrage, cela assure également une meilleure immobilisation primaire et assure une pression répartie uniformément sur toute la longueur de l'os.

De préférence, les arêtes sont sensiblement parallèles à l'axe X' X", cela contribue également à répartir uniformément la pression et diminue ainsi la douleur.

Lorsque les deux faces les plus proches des dièdres formant une paire d'arêtes passent sensiblement par l'axe, on obtient une meilleure résistance aux sollicitations en rotation puisque même si la tige s'enfonce dans l'os, elle reste entièrement en contact avec celui-ci sans que soient ménagées des zones vides, comme ce serait le cas sinon.

Pour assurer également une meilleure immobilisation, il est bon que, en vue en coupe transversalement à l'axe, les points correspondants aux arêtes soient sur un même cercle imaginaire. Toutes les arêtes sont alors efficaces en même temps.

On obtient une pression encore mieux répartie si chaque arête fait, au niveau d'une coupe transversale, un angle constant avec l'axe quelle que soit la coupe transversale.

Suivant l'invention, l'axe longitudinal de la zone d'ancrage, au lieu d'être rectiligne, est curviligne et s'étend suivant un arc de spirale logarithmique de forme :

$$ R \quad = \quad \left[ e^{k\theta} \right]_{\theta_{\text{début}}}^{\theta_{\text{fin}}} $$

R étant le rayon-vecteur, K le paramètre, $\theta_{\text{fin}}$ étant l'angle polaire du rayon-vecteur correspondant à la fin de l'arc sur la spirale et $\theta_{\text{début}}$ étant l'angle polaire du rayon-vecteur correspondant au début de l'arc sur la spirale, et les génératrices s'appuyant sur une directrice et passant par deux points par exemple diamétralement opposés de la directrice sont des arcs de spirale logarithmique, les spirales de l'axe curviligne et des deux génératrices ayant le même pôle et les arcs de spirale de l'axe et des deux génératrices ayant les mêmes $\theta f$ et $\theta d$, les longueurs des rayons-vecteurs des trois spirales étant, pour un même angle $\theta$, telles que R1 > R2 > R3, R2 étant le rayon vecteur de l'axe curviligne longitudinal et R1 et R3 ceux des deux génératrices considérées.

On obtient ainsi une zone d'ancrage incurvée qui peut mieux s'adapter à la paroi intérieure par exemple d'un fût fémoral et qui, néanmoins, permet de constituer une série optimisée de tiges d'ancrage, chaque tige ayant une zone de fixation identique, les tailles des zones d'ancrage des tiges de la série optimisée étant de plus en plus grandes et de plus en plus épaisses, caractérisée en ce que les zones d'ancrage de deux tiges consécutives de taille croissante sont telles que le $\theta$début de la première soit inférieur au $\theta$ début de la seconde et que le $\theta$fin de la première soit inférieur au $\theta$fin de la seconde, mais le $\theta$fin de la première est supérieur au $\theta$début de la seconde.

Il est ainsi possible de choisir et d'introduire dans le fût fémoral préparé, la tige qui convient pour que la partie de fixation se trouve à distance souhaitée de l'extrémité proximale du fût fémoral. On peut ainsi bien assembler la partie restante proximale de la prothèse, sans avoir à retailler le fût fémoral, puisque toutes les tiges de la série optimisée s'y adaptent bien.

La zone proximale peut être une zone de fixation sur laquelle s'adapte le module proximal. La zone proximale peut être aussi la zone qui tient lieu de module proximal, lorsque l'on a un composant prothétique monobloc.

Aux dessins annexés, donnés uniquement à titre d'exemple :

- la figure 1 est une vue en perspective d'une tige d'ancrage suivant l'invention
- la figure 2 est une vue en coupe transversale de la zone d'ancrage de la tige de la figure 1

- la figure 3 est un schéma illustrant une tige d'ancrage suivant l'invention
- la figure 4 illustre une série optimisée de tiges d'ancrage suivant l'invention, et
- la figure 5 illustre le montage d'une tige dans un fût fémoral préparé
- la figure 6 est un schéma illustrant la manière de réaliser une série optimisée de tiges d'ancrage suivant l'invention.

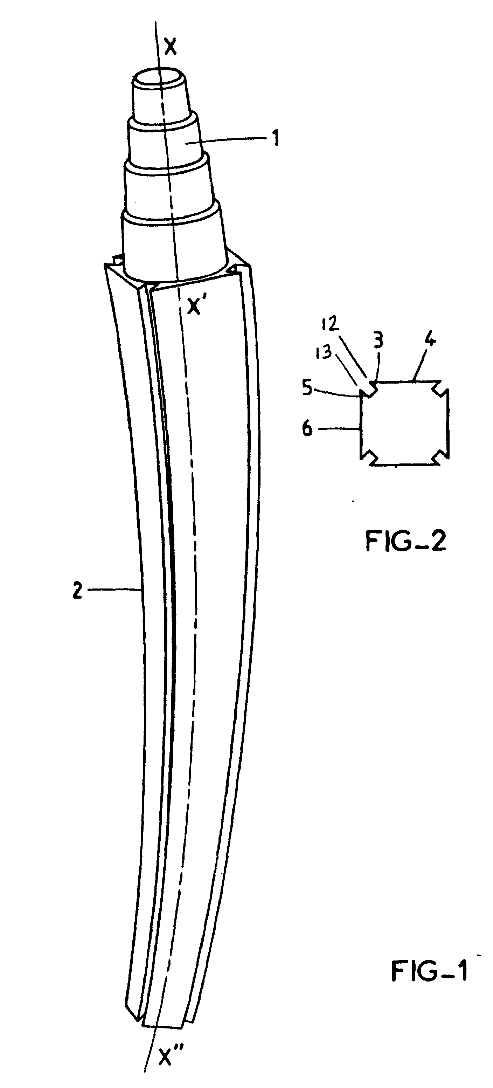

La tige d'ancrage représentée aux figures 1 et 2 comprend une zone proximale 1 constituée de quatre cônes superposés d'axe XX' prolongés d'une zone d'ancrage 2 d'axe curviligne X'X''.

La section transversale de la zone d'ancrage 2 est sensiblement carrée pour cet exemple simplifié; les côtés du carré faisant donc entre eux un angle de 90°, sauf que, à chaque sommet 12, 13 correspondant aux arêtes, il est ménagé un renfoncement selon l'invention. Le côté 3 du renfoncement adjacent à un côté 4 de la section transversale fait avec celui-ci un angle aigu inférieur à 60°, donc tranchant.

Les deux côtés 3, 5 d'un même renfoncement font entre eux un angle de 0°, le renfoncement ne s'évase donc pas vers l'intérieur. Dans un autre exemple, non représenté, le renfoncement peut s'évaser vers l'extérieur.

De même, le côté 5 du renfoncement adjacent au côté 6 de la section transversale constitue aussi un tranchant.

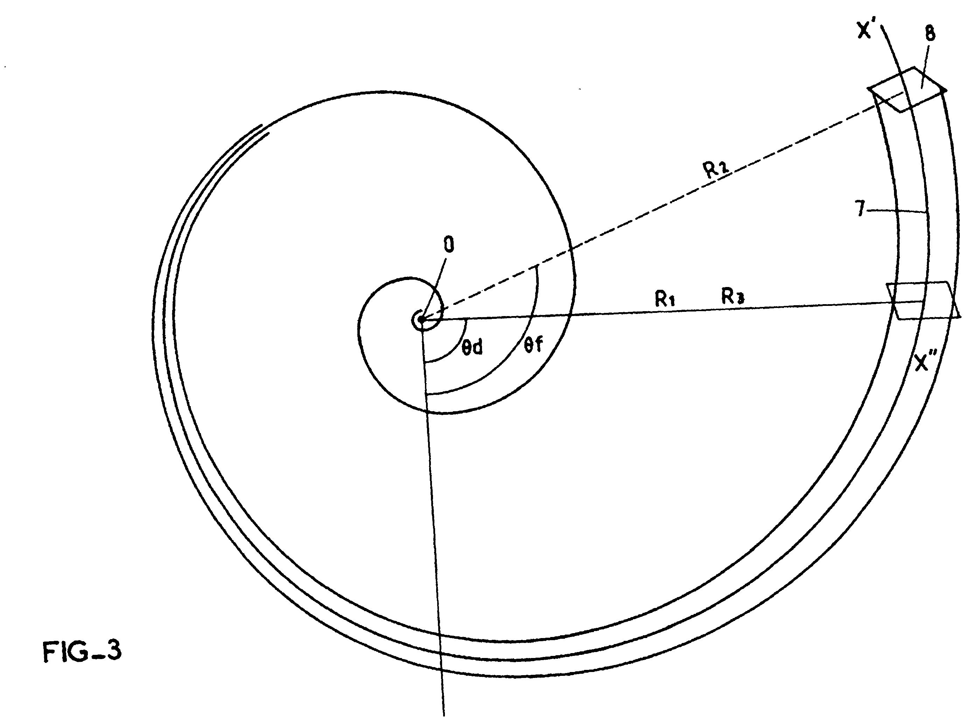

L'axe X'X'' longitudinal curviligne de la zone d'ancrage 2 s'étend suivant un arc 7 (figure 3) de spirale logarithmique de formule :

$$R2 \quad = \quad \left[ e^{k2\theta} \right]_{\theta d\acute{e}but}^{\theta fin}$$

R2 étant le rayon-vecteur, K2 le paramètre, θfin l'angle du rayon-vecteur correspondant à la fin de l'arc sur la spirale deθdébut l'angle du rayon-vecteur correspondant au début de l'arc sur la spirale. De la même façon, les génératrices 8 s'appuyant sur la directrice 9 qui, en l'espèce, est carrée et correspond à la section transversale de la tige, sont des arcs de spirales logarithmiques de rayons-vecteurs R1 et R3, les trois spirales logarithmiques ayant un pôle commun 0 et R1 > R2 > R3, K1, K2, K3 étant choisis en conséquence.

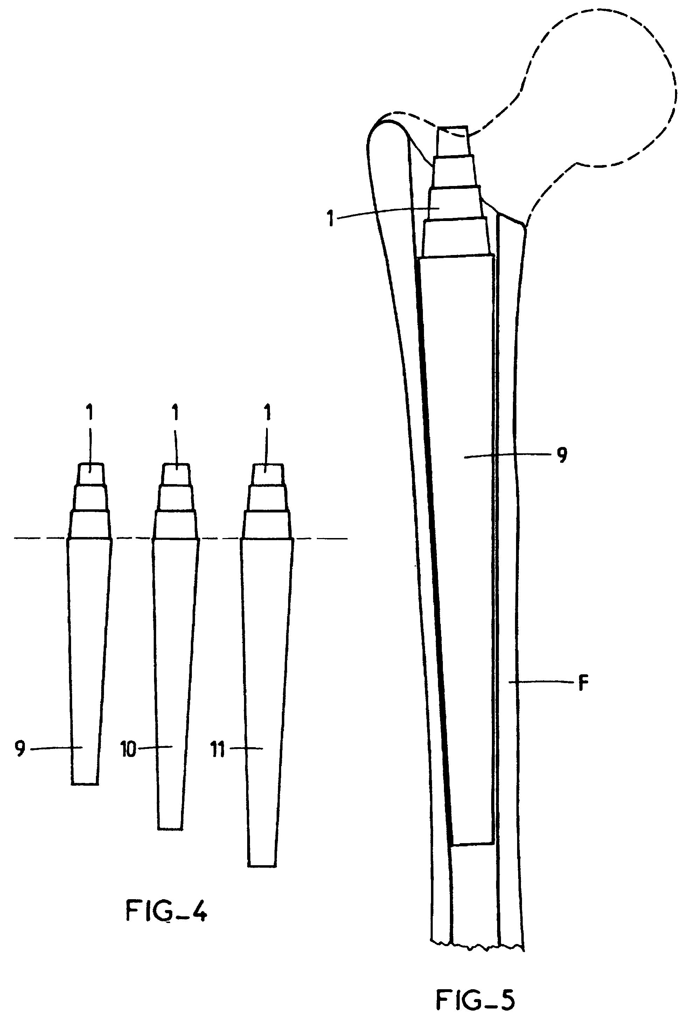

La figure 4 représente trois tiges 10, 11, 12, d'une série optimisée de tiges d'ancrage.

Les zones d'ancrage sont de plus en plus grandes et de plus en plus épaisses de la tige 10 à la tige 12, alors que les zones de fixation sont identiques. Les zones d'ancrage de deux tiges 10, 11, consécutives de taille croissante sont telles que le θdébut de la tige 10 soit inférieur au θdébut de la tige 11, mais le θfin de la tige 10 est supérieur au θdébut de la tige 11.

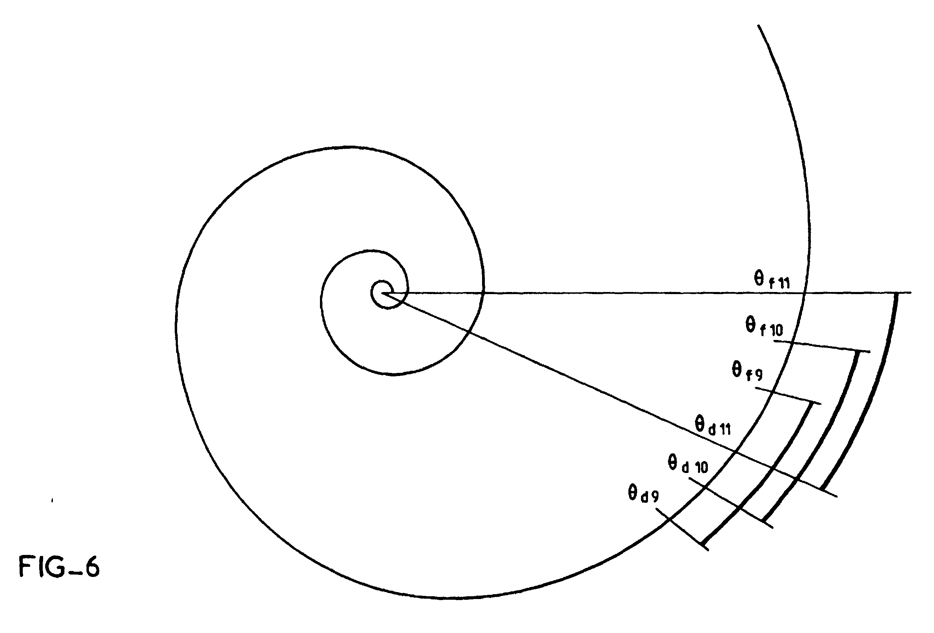

Les arcs de spirales logarithmiques pris pour constituer les trois tiges de la série sont schématisés à la figure 6.

La figure 5 montre que l'on peut insérer l'une quelconque des tiges, par exemple la tige 10 dans le fût fémoral F en sorte que la zone de fixation se trouve en position souhaitée pour recevoir le module proximal d'une prothèse.

**Revendications**

1. Tige d'ancrage comportant une zone proximale (1) et une zone d'ancrage (2) d'axe (X'X''), qui a au moins deux paires d'arêtes (12, 13 et 14, 15) tranchantes, chaque arête étant formée d'un dièdre, les deux faces les plus éloignées des dièdres de chaque paire d'arêtes faisant entre elles un angle égal ou supérieur à 60° et égal ou inférieur à 120°, les directions des faces n'étant considérées que sur une distance de l'ordre du millimètre à partir des arêtes, les arêtes s'étendant sensiblement sur toute la longueur de la zone d'ancrage (2), l'axe (X'X'') longitudinal de la zone d'ancrage étant curviligne, **caractérisée en ce que** ladite axe de la zone d'ancrage s'etend suivant un arc de spirale logarithmique de formule

$$R = \quad \left[ e^{k\theta} \right]_{\theta d\acute{e}but}^{\theta fin}$$

R étant le rayon-vecteur, k le paramètre, θfin étant l'angle polaire du rayon-vecteur correspondant à la fin de l'arc sur la spirale et θdébut étant l'angle polaire du rayon-vecteur correspondant au début de l'arc sur la spirale, et les génératrices s'appuyant sur une directrice et passant par deux points de part et d'autre de l'axe de directrice sont des arcs de spirale logarithmique, les spirales de l'axe curviligne et des deux génératrices ayant le même pôle et les arcs de spirale de l'axe et des deux génératrices ayant les mêmes θf et θd, les longueurs des rayons vecteurs des trois spirales étant, pour un même angle θ, telles que R1 > R2 > R3, R2 étant le rayon-vecteur de l'axe curviligne longitudinal et R1 et R3 ceux des deux génératrices.

2. Tige d'ancrage suivant fa revendication 1, **caractérisée en ce que** les arêtes sont sensiblement parallèles à l'axe (X'X").

3. Tige d'ancrage suivant l'une. quelconque des revendications précédentes, **caractérisée en ce que** les deux faces les plus proches des dièdres formant une paire d'arêtes passent sensiblement par l'axe (X'X").

4. Tige d'ancrage suivant l'une quelconque des revendications précédentes, **caractérisée en ce que**, en vue en coupe transversalement à l'axe (X'X"), les points correspondants aux arêtes sont sur un même cercle imaginaire.

5. Tige d'ancrage suivant l'une quelconque des revendications précédentes, **caractérisée en ce que** chaque arête fait, au niveau d'une coupe transversale, un angle constant avec l'axe (X'X"), quelle que soit la coupe transversale.

6. Série optimisée de tiges d'ancrage suivant la revendication 1, chaque tige ayant une zone de fixation identique, les tailles des zones d'ancrage des tiges de la série étant dé plus en plus grandes et de plus en plus épaisses, **caractérisée en ce que** les zones d'ancrage de deux tiges consécutives de taille croissante sont telles que le θdébut de la première soit inférieur au θdébut de la seconde et que le θfin de la première soit inférieur au θfin de la seconde, mais le θfin de la première est supérieur au θdébut de la seconde.


**Patentansprüche**

1. Verankerungsstange, die eine proximale Zone (1) und eine Verankerungszone (2) der Achse (X'X") beinhaltet, die mindestens zwei Paar scharfe Kanten (12, 13 und 14, 15) aufweist, wobei jede Kante durch einen Dieder geformt wird und die zwei Flächen, die am weitesten von den Diedern jedes Kantenpaars entfernt sind, untereinander einen Winkel bilden, der gleich oder größer ist als 60° und gleich oder kleiner ist als 120° beträgt, wobei die Richtungen der Fläche nur auf einer millimetergroßen Distanz ab den Kanten betrachtet werden und die Kanten sich im Wesentlichen auf die ganze Länge der Verankerungszone (2) erstrecken, wobei die Längsachse (X'X") der Verankerungszone krummlinig ist und sich die genannte Achse der Verankerungszone gemäß einem logarithmischen Spiralbogen erstreckt, der folgende Formel hat:

$$R = \left[ e^{k\theta} \right]_{\theta Anfang}^{\theta Ende}$$

wobei R der Radiusvektor ist, k der Parameter, θEnde der polare Winkel des Radiusvektors am Ende des Bogens auf der Spirale ist und θAnfang der polare Winkel des Radiusvektors am Anfang des Bogens auf der Spirale ist, und die Mantellinien, die sich auf eine Leitlinie stützen und durch zwei Punkte auf beiden Seiten der Leitachse führen, Bögen einer logarithmischen Spirale sind, wobei die Spiralen der krummlinigen Achse und der zwei Mantellinien denselben Pol haben und die Spiralbögen der Achse und der zwei Mantellinien die selben θE und θA haben, die Längen der Vektorradien der drei Spiralen für einen gleichen Winkel θ so sind wie R1 > R2 > R3, wobei R2 der Vektorradius der krummlinigen Längenachse ist und R1 und R3 die der beiden Mantellinien.

2. Verankerungsstange gemäß Anspruch 1, **dadurch gekennzeichnet, dass** die Kanten im Wesentlichen parallel zur Achse (X'X") laufen.

3. Verankerungsstange gemäß einem der vorangegangen Ansprüche, **dadurch gekennzeichnet, dass** die zwei den Diedern am nächsten gelegenen Flächen, die ein Kantenpaar bilden, im Wesentlichen durch die Achse (X'X") verlaufen.

4. Verankerungsstange gemäß einem der vorangegangen Ansprüche,
**dadurch gekennzeichnet, dass** bei einer quer zur Achse (X'X") verlaufenden Schnittansicht die den Kanten entsprechenden Punkte auf einem gleichen imaginären Kreis liegen.

5. Verankerungsstange gemäß einem der vorangegangen Ansprüche,
**dadurch gekennzeichnet, dass** jede Kante auf der Ebene eines Querschnitts einen konstanten Winkel mit der Achse (X'X") bildet, egal welcher Querschnitt betrachtet wird.

6. Optimierte Serie von Verankerungsstangen gemäß Anspruch 1, wobei jede Stange eine identische Fixierungszone besitzt, wobei die Form der Verankerungszonen der Stangen der Serie immer größer und dicker wird,
**dadurch gekennzeichnet, dass** die Verankerungszonen von zwei konsekutiven Stangen mit größer werdender Form so sind, dass θAnfang der ersten kleiner ist als θAnfang der zweiten und dass θEnde der ersten kleiner ist als θEnde der zweiten, aber θEnde der ersten größer ist als θAnfang der zweiten.

## Claims

1. Anchoring rod comprising a proximal zone (1) and an anchoring zone (2) having an axis (X' X"), the anchoring zone having at least two pairs of cutting fins (12, 13 and 14, 15) each fin being formed by a dihedron, the two most remote sides of the dihedra of each pair of fins forming between them an angle equal to or greater than 60° and equal to or less than 120°, the directions of the sides being taken into consideration only over a distance of the order of one millimetre from the fins, wherein the fins extend substantially over the entire length of the anchoring zone (2), wherein the longitudinal axis (X, X") of the anchoring zone is curved, **characterized in that** said axis of the anchoring zone extends along a logarithmic spiral arc of formula :

$$R = \left[ e^{k\theta} \right]_{\theta_{start}}^{\theta_{finish}}$$

where R is the vector radius, K is the parameter, $\theta_{finish}$ is the polar angle of the vector radius corresponding to the end of the arc on the spiral and $\theta_{start}$ is the polar angle of the vector radius corresponding to the start of the arc on the spiral, and the generatrices bearing on one directrix and passing through two points on either side of the directrix are logarithmic spiral arcs, the spirals of the curved axis and the two generatrices having the same pole and the spiral arcs of the axis and the two generatrices having the same $\theta f$ and $\theta s$, the lengths of the vector radii of the three spirals being, for the same angle $\theta$, such that R1>R2>R3, R2 being the vector radius of the longitudinal curved axis and R1 and R3 being those of the two generatrices.

2. Anchoring rod according to claim 1, **characterized in that** the fins are substantially parallel to the axis (X', X").

3. Anchoring rod according to anyone of the previous claims , **characterized in that** the two closest sides of the dihedra are forming a pair of fins passing substantially through the axis (X, X").

4. Anchoring rod according to anyone of the previous claims, **characterized in that**, viewed in section transversally to the axis (X', X"), the points corresponding to the fins are on the same imaginary circle.

5. Anchoring rod according to anyone of the previous claims, **characterized in that** in cross section, each fin forms a constant angle with the axis (X', X"), no matter where the section is taken.

6. Optimised series of anchoring rods according to claim 1, each rod having an identical fixing zone, the sizes of the anchoring zones of the rods in the series being progressively larger and progressively thicker, **characterized in that** the anchoring zones of two consecutive rods of increasing size are such that the $\theta_{start}$ of the first is greater than the $\theta_{start}$ of the second and the $\theta_{finish}$ of the first is less than the $\theta_{finish}$ of the second, but the $\theta_{finish}$ of the first is greater than the $\theta_{start}$ of the second

EP 0 790 045 B1

FIG-2

FIG-1

FIG-3

EP 0 790 045 B1

FIG_4

FIG_5

FIG-6

EP 0 790 045 B1